# EUROPEAN PATENT SPECIFICATION

(11) **EP 4 046 618 B1**
(45) Date of publication and mention of the grant of the patent: **22.04.2026**
(21) Application number: 20876872.1
(22) Date of filing: 07.07.2020
(51) Int. Cl.: A61K 6/90, A61K 6/70

(54) **PASTE CURING MATERIAL AND DENTAL ALGINATE IMPRESSION MATERIAL**
PASTEN-AUSHÄRTUNGSMATERIAL UND ALGINAT-DENTALABFORMMASSE
MATÉRIAU DE DURCISSEMENT DE PÂTE ET MATÉRIAU D'EMPREINTE DENTAIRE À BASE D'ALGINATE

(30) Priority: 17.10.2019 JP 2019190243
(43) Date of publication of application: 24.08.2022
(73) Proprietor: GC Corporation, Sunto-gun, Shizuoka 410-1307 (JP)
(72) Inventor: NAKASHIMA, Yusuke, Tokyo 174-8585 (JP); NIIZEKI, Naofumi, Tokyo 174-8585 (JP)
(74) Representative: Müller-Boré & Partner Patentanwälte PartG mbB
(86) International application number: PCT/JP2020/026501
(87) International publication number: WO 2021/075095

(56) References cited:
- EP-A1- 2 638 893
- WO-A1-2012/063618
- WO-A1-2014/050028
- WO-A1-2017/163491
- CN-A- 110 051 542

## Description

### [Technical Field]

The present invention relates to a paste curing material used for a dental alginate impression material, and a dental alginate impression material.

### [Background Art]

In dental treatment, an alginate impression material is widely used as an impression material for taking an impression of an oral cavity at the time of producing a prosthesis.

As an alginate impression material, an alginate impression material is known that includes a paste base material containing alginate and water, and a paste curing material containing a gelatinizing reactant and a poorly water-soluble liquid compound (see for example, Patent Literature 1-3).

### [Citation List]

### [Patent Literature]

[PTL 1]
   WO 2012/063618
[PTL 2]
   WO 2014/050028 PP
[PTL 3] CN 110 051 542 A

### [Summary of Invention]

### [Technical Problem]

However, there was a problem that the paste curing material tends to be separated into solid and liquid, and has low storage stability.

One aspect of the present invention is to provide a paste curing material having excellent storage stability.

### [Solution to Problem]

One aspect of the present invention includes: a paste curing material used for dental alginate impression. The paste curing material includes a gelatinizing reactant; a poorly water-soluble liquid compound; and a smectite.

### [Advantageous Effects of Invention]

According to one aspect of the present invention, a paste curing material having excellent storage stability can be provided.

### [Description of Embodiments]

In the following, embodiments of the present invention will be described.

### [Paste curing material]

A paste curing material according to the present embodiment is used for a dental alginate impression material according to the present embodiment described later.

The paste curing material according to the present embodiment contains a gelatinizing reactant, a poorly water-soluble liquid compound, and a smectite.

As the gelatinizing reactant, a compound of a metal having two or more valence can be used. For example, calcium sulfates such as calcium sulfate dihydrate (CaSO₄· 2H₂O), calcium sulfate hemihydrate (CaSO₄· 1/2H₂O), anhydrous calcium sulfate (CaSO₄), and the like can be used.

Examples of the gelatinizing reactant other than the above include oxides, hydroxides, and the like of a metal having two or more valence such as calcium, magnesium, zinc, aluminum, iron, titanium, zirconium, tin, and the like.

Examples of the oxides of a metal having two or more valence include calcium oxide, magnesium oxide, zinc oxide, titanium oxide, zirconium oxide, tin oxide, and the like.

Examples of the hydroxides of a metal having two or more valence include calcium hydroxide, magnesium hydroxide, zinc hydroxide, aluminum hydroxide, iron hydroxide, and the like.

Two or more gelatinizing reactants may be used in combination.

The content of the gelatinizing reactant in the paste curing material according to the present embodiment is preferably 10 to 60% by mass. When the content of the gelatinizing reactant in the paste curing material according to the present embodiment is 10% by mass or more, the strength of the cured product of the dental alginate impression material according to the present embodiment is improved, and when the content is 60% by mass or less, the open time of the dental alginate impression material according to the present embodiment is increased.

As used herein and in the claims, "poorly water-soluble" means having a solubility of 5 g or less in 100 g of water at 20°C.

The poorly water-soluble liquid compound is not particularly limited as long as it permits the gelatinizing reactant to form a paste. Examples of the poorly water-soluble liquid compound include hydrocarbon compounds, aliphatic alcohols, aromatic alcohols, fatty acids, fatty acid salts, fatty acid esters, silicone oils, and the like.

Examples of the hydrocarbon compound include aliphatic chain hydrocarbon compounds such as hexane, heptane, octane, nonane, decane, undecane, dodecane, tridecane, tetradecane, pentadecane, kerosene, 2,7-dimethyl octane, 1-octene, and the like; alicyclic hydrocarbon compounds such as cycloheptane, cycloncnane, and the like; liquid paraffins, and the like.

Examples of the aliphatic alcohol include saturated aliphatic alcohols such as 1-hexanol, 1-octanol, and the like, and unsaturated aliphatic alcohols such as citronellol, oleyl alcohols, and the like.

Examples of the aromatic alcohol include benzyl alcohol, m-cresol, and the like.

Examples of the fatty acid include saturated fatty acids such as hexanoic acid, octanoic acid, and the like, and unsaturated fatty acids such as oleic acid, linoleic acid, and the like.

Examples of the fatty acid ester include ethyl octanoate, butyl phthalate, glyceride oleate, vegetable oils such as olive oil and sesame oil, animal fats such as liver oil and whale oil, and the like.

Examples of the silicone oil include polydimethylsiloxane, polymethylphenylsiloxane, polymethylhydrogensiloxane, polyphenylhydrogensiloxane, and the like.

Two or more poorly water-soluble liquid compounds may be used in combination.

The content of the poorly water-soluble liquid compound in the paste curing material according to the present embodiment is preferably 10 to 50% by mass, and further preferably 15 to 30% by mass. When the content of the poorly water-soluble liquid compound in the paste curing material according to the present embodiment is 10% by mass or more, the handleability of the dental alginate impression material according to the present embodiment is improved, and when the content is 50% by mass or less, the strength of the cured product of the dental alginate impression material according to the present embodiment is improved.

Examples of the smectite include montmorillonite, bentonite, beidellite, nontronite, saponite, hectorite, sauconite, stevensite, and the like.

Two or more smectites may be used in combination.

The content of the smectite in the paste curing material according to the present embodiment is preferably 0.1 to 50% by mass, and further preferably 0.5 to 5% by mass. When the content of smectite in the paste curing material according to the present embodiment is 0.1% by mass or more, the storage stability of the paste curing material according to the present embodiment is improved, and when the content is 50% by mass or less, the handleability of the dental alginate impression material according to the present embodiment is improved.

The paste curing material according to the present embodiment preferably further includes polybutene. Accordingly, the storage stability of the paste curing material according to the present embodiment is improved. Also, the working time of the dental alginate impression material according to the present embodiment is increased.

The polybutene is preferably a liquid polymer.

The polybutene may be synthesized, for example, by copolymerizing isobutene with 1-butene.

The number average molecular weight of the polybutene is preferably 300 to 4000.

The content of the polybutene in the paste curing material according to the present embodiment is preferably 0.5 to 30% by mass, and further preferably 1 to 20% by mass. When the content of the polybutene in the paste curing material according to the present embodiment is 0.5% by mass or more and 30% by mass or less, the handleability of the dental alginate impression material according to the present embodiment is improved.

The paste curing material according to the present embodiment preferably further includes a retarder. Accordingly, the working time of the dental alginate impression material according to the present embodiment is increased.

Examples of the retarder include sodium pyrophosphate, potassium pyrophosphate, and the like.

Two or more retarders may be used in combination.

The content of the retarder in the paste curing material according to the present embodiment is preferably 0.01 to 10% by mass. When the content of the retarder in the paste curing material according to the present embodiment is 0.01% by mass or more, the working time of the dental alginate impression material according to the present embodiment is increased, and when the content is 10% by mass or less, the initial setting time of the dental alginate impression material according to the present embodiment is shortened, and the strength of the cured product of the dental alginate impression material according to the present embodiment is improved.

The paste curing material according to the present embodiment may further include curing modifiers, surfactants, fillers, and the like.

Examples of the curing modifier include potassium titanium fluoride, magnesium oxide, magnesium hydroxide, zinc oxide, and the like.

Examples of the surfactant include polyoxyethylene alkyl ether and the like.

Examples of the filler include inorganic oxides such as silica and alumina, and the like.

Two or more curing accelerators, the surfactants, and the fillers may be used in combination.

### [Dental alginate impression material]

The dental alginate impression material according to the present embodiment includes a paste base material and the paste curing material according to the present embodiment.

The paste base material contains an alginate and water.

Examples of the alginate include alkali metal salts of alginates such as sodium alginate, potassium alginate, and the like; and ammonium salts of alginates such as ammonium alginate, triethanolamine alginate, and the like. Among these, the alkali metal salts of alginates are preferable from the viewpoint of accessibility, ease of handling, physical properties of the cured product of the dental alginate impression material according to the present embodiment, and the like.

Two or more alginates may be used in combination.

The alginate content in the paste base material is preferably 1 to 20% by mass.

The water content in the paste base material is preferably 80 to 99% by mass.

The paste base material may further contain a retarder. Accordingly, the working time of the dental alginate impression material according to the present embodiment is increased.

Examples of the retarder include sodium pyrophosphate, potassium pyrophosphate and the like.

Two or more retarders may be used in combination.

The content of the retarder in the paste base material is preferably 0.01 to 10% by mass. When the content of the retarder in the paste base material is 0.01% by mass or more, the working time of the dental alginate impression material according to the present embodiment is increased, and when the content is 10% by mass or less, the initial setting time of the dental alginate impression material according to the present embodiment is shortened, and the strength of the cured product of the dental alginate impression material is improved.

The dental alginate impression material according to the present embodiment may further include colorants, preservatives, disinfectants, perfumes, pH modifiers, and the like.

### [Method of using dental alginate impression material]

The dental alginate impression material according to the present embodiment is used by kneading the paste base material and the paste curing material.

The mass ratio of the paste base material to the paste curing material in the kneading of the paste curing material and the paste base material is preferably 1 to 5.

The content of the gelatinizing reactant in the kneaded product of the paste curing material and the paste base material is preferably 2 to 30% by mass.

The content of the poorly water-soluble liquid compound in the kneaded product of the paste curing material and the paste base material is preferably 2 to 25% by mass.

The content of the smectite in the kneaded product of the paste curing material and the paste base material is preferably 0.02 to 25% by mass.

The content of the polybutene in the kneaded product of the paste curing material and the paste base material is preferably 0.1 to 15% by mass.

The content of the retarder in the kneaded product of the paste curing material and the paste base material is preferably 0.002 to 5% by mass.

The content of the alginate in the kneaded product of the paste curing material and the paste base material is preferably 0.3 to 18% by mass.

The content of water in the kneaded product of the paste curing material and the paste base material is preferably 25 to 85% by mass.

### [Example]

Hereinafter, examples of the present invention will be described, but the present invention is not limited to the examples.

### [Preparation of paste base material]

A paste base material was obtained by mixing 10% by mass of potassium alginate, 89.8% by mass of distilled water, and 0.2% by mass of sodium pyrophosphate.

### [Preparation of paste curing material]

A paste curing material was obtained by mixing a gelatinizing reactant, a poorly water-soluble liquid compound, smectite, polybutene, a retarder, a curing accelerator, a surfactant, and a filler in the formulation (% by mass) illustrated in Table 1.

The details of the smectite are as follows.

Montmorillonite: Kunipia-F (manufactured by KUNIMINE INDUSTRIES CO., LTD.)
Bentonite: Moistnite-U (manufactured by KUNIMINE INDUSTRIES CO., LTD.)
Hectorite: Sumecton-SWN (manufactured by KUNIMINE INDUSTRIES CO., LTD.)
Stevensite: Sumecton-ST (manufactured by KUNIMINE INDUSTRIES CO., LTD.)

Next, the storage stability of the paste curing material was evaluated.

### [Storage stability of paste curing material]

The paste curing material was filled into a 5 ml glass bottle, and then left at 60°C for 1 week. The storage stability of the paste curing material was evaluated by visually observing the degree of solid-liquid separation.

The storage stability of the paste curing material was determined according to the following criteria.

Excellent: Solid-liquid separation was not observed.
Bad: Solid-liquid separation was observed.

Next, the initial curing time of the dental alginate impression material was evaluated.

### [Initial setting time of dental alginate impression material]

The paste curing material and the paste base material were kneaded at a mass ratio of 1 : 2. The initial curing time of the dental alginate impression material was measured according to "7.2 initial setting time test" of JIS T 6505:2016 "Dental alginate impression materials".

When the initial setting time of the dental alginate impression material is 30 seconds or more, the working time is long, and when the initial setting time is 4 minutes or less, the initial setting time is short. Accordingly, the dental alginate impression material becomes appropriate.

Table 1 illustrates the evaluation results of the storage stability of the paste curing material and the initial setting time of the alginate impression material.

**[Table 1]**

| | EXAMPLE | | | | | COMPARATIVE EXAMPLE |
|---|---|---|---|---|---|---|
| | 1 | 2 | 3 | 4 | 5 | 1 |
| FORMULATION OF HARDENING MATERIAL PASTE | | | | | | |
| CALCIUM SULFATE HEMIHYDRATE | 40 | 40 | 40 | 40 | 40 | 40 |
| ANHYDROUS CALCIUM SULFATE | 5 | 5 | 5 | 5 | 5 | 8 |
| MAGNESIUM HYDROXIDE | 5 | 5 | 5 | 5 | 5 | 5 |
| LIQUID PARAFFIN | 20 | 20 | 20 | 20 | 23 | 20 |
| MONTMORILLONITE | 3 | | | | 3 | |
| BENTONITE | | 3 | | | | |
| HECTORITE | | | 3 | | | |
| STEVENSITE | | | | 3 | | |
| POLYBUTENE | 3 | 3 | 3 | 3 | | 3 |
| SODIUM PYROPHOSPHATE | 1 | 1 | 1 | 1 | 1 | 1 |
| POTASSIUM TITANIUM FLUORIDE | 5 | 5 | 5 | 5 | 5 | 5 |
| POLYOXYETHYLENE ALKYL ETHER | 3 | 3 | 3 | 3 | 3 | 3 |
| SILICA | 15 | 15 | 15 | 15 | 15 | 15 |
| TOTAL | 100 | 100 | 100 | 100 | 100 | 100 |
| EVALUATION RESULT | | | | | | |
| STORAGE STABILITY | EXCELLENT | EXCELLENT | EXCELLENT | EXCELLENT | EXCELLENT | BAD |
| INITIAL SETTING TIME (SEC) | 120 | 120 | 90 | 145 | 120 | 105 |

From Table 1, it can be seen that the paste curing materials of Examples 1 to 5 have high storage stability.

In contrast, the paste curing material of Comparative Example 1 does not contain smectite, and thus has low storage stability.

## Claims

1. A paste curing material used for dental alginate impression, the paste curing material comprising:
a gelatinizing reactant;
a poorly water-soluble liquid compound, wherein "poorly water-soluble" means having a solubility of 5 g or less in 100 g of water at 20°C; and
a smectite;
wherein the gelatinizing reactant is a compound of a metal having a valence of two or more, and
the content of the smectite in the paste curing material is 0.1 to 50% by mass.

2. The paste curing material according to claim 1, wherein the smectite is montmorillonite, bentonite, beidellite, nontronite, saponite, hectorite, sauconite, or stevensite.

3. A dental alginate impression material comprising:
an alginate;
a paste base material containing a water; and
the paste curing material of claim 1.

## Patentansprüche

1. Pastenaushärtungsmaterial, das für dentale Alginatabdrücke verwendet wird, wobei das Pastenaushärtungsmaterial umfasst:
einen gelierenden Reaktanten;
eine schwer wasserlösliche flüssige Verbindung, wobei "schwer wasserlöslich" das Aufweisen einer Löslichkeit von 5 g oder weniger in 100 g Wasser bei 20 °C bedeutet; und
ein Smektit;
wobei der gelierende Reaktant eine Verbindung eines Metalls mit einer Valenz von zwei oder mehr ist und
der Gehalt an dem Smectit in dem Pastenaushärtungsmaterial 0,1 bis 50 Massen-% beträgt.

2. Pastenaushärtungsmaterial nach Anspruch 1, wobei der Smectit Montmorillonit, Bentonit, Beidellit, Nontronit, Saponit, Hectorit, Sauconit oder Stevensit ist.

3. Dentales Alginatabdruckmaterial, umfassend:
ein Alginat;
ein Pastenbasismaterial, das Wasser enthält; und
das Pastenaushärtungsmaterial nach Anspruch 1.

## Revendications

1. Matériau de durcissement de pâte utilisé pour une empreinte dentaire à base d'alginate, le matériau de durcissement de pâte comprenant :
un réactif gélifiant ;
un composé liquide faiblement soluble dans l'eau, dans lequel « faiblement soluble dans l'eau » signifie ayant une solubilité de 5 g ou moins dans 100 g d'eau à 20 °C ; et
une smectite ;
dans lequel le réactif gélifiant est un composé d'un métal ayant une valence de deux ou plus, et
la teneur en smectite du matériau de durcissement de pâte est de 0,1 à 50 % en masse.

2. Matériau de durcissement de pâte selon la revendication 1, dans lequel la smectite est la montmorillonite, la bentonite, la beidellite, la nontronite, la saponite, l'hectorite, la sauconite ou la stevensite.

3. Matériau d'empreinte dentaire à base d'alginate comprenant :
un alginate ;
un matériau à base de pâte contenant de l'eau ; et
le matériau de durcissement de pâte selon la revendication 1.
